(19) Europäisches Patentamt
European Patent Office
Office européen des brevets

(11) EP 4 722 156 A1

(12) EUROPEAN PATENT APPLICATION
published in accordance with Art. 153(4) EPC

(43) Date of publication:
**08.04.2026 Bulletin 2026/15**

(21) Application number: **24811103.1**

(22) Date of filing: **21.05.2024**

(51) International Patent Classification (IPC):
***C01B 33/141*** *(2006.01)* ***B01J 13/00*** *(2006.01)*
***C07D 275/03*** *(2006.01)* ***C07D 275/04*** *(2006.01)*
***C09K 3/14*** *(2006.01)* ***H01L 21/304*** *(2006.01)*

(52) Cooperative Patent Classification (CPC):
**B01J 13/00; C01B 33/141; C07D 275/03; C07D 275/04; C09K 3/14; H10P 52/00**

(86) International application number:
**PCT/JP2024/018642**

(87) International publication number:
**WO 2024/242096 (28.11.2024 Gazette 2024/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.05.2023 JP 2023085593**

(71) Applicant: **SHIN-ETSU CHEMICAL CO., LTD.**
**Tokyo 100-0005 (JP)**

(72) Inventor: **MATSUMURA, Kazuyuki**
**Annaka-shi, Gunma 379-0224 (JP)**

(74) Representative: **Angerhausen, Christoph**
**Boehmert & Boehmert**
**Anwaltspartnerschaft mbB**
**Pettenkoferstrasse 22**
**80336 München (DE)**

# (54) COLLOIDAL SILICA AND METHOD FOR PRODUCING SAME

(57) Provided are a colloidal silica that maintains low viscosity even at high concentration and exhibits minimal viscosity increase during long-term storage and a method for producing the same. The colloidal silica contains silica particles, at least one dispersant selected from 1,2-benzisothiazolin-3-one and isothiazolin-3-one, and water.



Processed by Luminess, 75001 PARIS (FR)

## Description

### Technical Field

The present invention relates to a colloidal silica and a method for producing the same.

### Background Art

With the remarkable miniaturization of semiconductor devices in recent years, colloidal silica, having small silica particles, has been employed in processes such as silicon wafer polishing and chemical mechanical polishing (hereinafter referred to as "CMP") of semiconductor devices.

High-purity colloidal silica with bare minimal metal impurities has been highly sought after as a colloidal silica for CMP processes, the reason being to prevent contamination, particularly of silicon wafers. Patent document 1 discloses a method for producing colloidal silica that has little content of metal impurities and is modified by a silane coupling agent having a cationic group. However, the cationic groups present on the surface of silica occasionally form hydrogen bonds with silanol groups, or the cationic groups act as condensation catalysts between silanol groups, resulting in aggregation of silica particles by themselves.

It is also preferred to manufacture colloidal silica with a high silica concentration, as this not only enhances production efficiency but also optimizes storage and transportation efficiency, permits flexibility in the adjustment of abrasive blends utilized in polishing, and increases the degree of freedom in formulation.

However, when a colloidal silica is used in a CMP process, reuse of the colloidal silica leads to an increase in viscosity of the colloidal silica, leading to an issue that the effectiveness as an abrasive cannot be fully demonstrated. Accordingly, there exists a demand for highly concentrated colloidal silica that demonstrates minimal viscosity increase over a prolonged period of time.

In this regard, patent documents 2 and 3 disclose methods of adding an inorganic or organic acid dispersant, such as sulfuric acid, nitric acid, or citric acid, to colloidal silica to improve the dispersibility of highly concentrated colloidal silica. These dispersants may be used to prevent the introduction of metal impurities while enhancing the dispersibility of colloidal silica. However, prolonged storage of them can lead to pH changes, which occasionally cause the silicas to aggregate by themselves or exhibit increased viscosity.

Patent document 4 discloses a method, comprising: treating a part of the surface of water-dispersed colloidal silica, obtained by a sol-gel method in the presence of a hydrophilic organic solvent, with a tri-functional silane compound or the like; and subsequently substituting the dispersion medium with water, thereby producing a water-dispersed surface-treated colloidal silica that contains no metal impurities or dispersants, is highly concentrated, and exhibits minimal viscosity increase over a long period. However, this method requires a surface treatment of silica, and therefore, a simpler method has been sought after.

### Prior Art Documents

### Patent documents

Patent document 1: JP-A-2005-162533
Patent document 2: JP-B-5270344
Patent document 3: JP-A-2019-172558
Patent document 4: JP-A-2023-005414

### Summary of Invention

### Technical Problem

It is therefore an object of the present invention to provide a colloidal silica that maintains low viscosity even at high concentration and exhibits minimal viscosity increase during long-term storage.

### Solution to Problem

The present inventor has diligently studied to solve the above objects and, as a result, has discovered that a use of 1,2-benzisothiazolin-3-one and/or isothiazolin-3-one as a dispersant results in a colloidal silica that maintains low

viscosity even at high concentration and exhibits minimal viscosity increase during long-term storage without requiring any surface treatment of silica, thereby completing the present invention.

That is, the present invention intends to provide a colloidal silica and a method for producing the colloidal silica, which are as set forth below:

<1> A colloidal silica comprising:

silica particles;
at least one dispersant selected from 1,2-benzisothiazolin-3-one and isothiazolin-3-one; and
water.

<2> The colloidal silica according to <1>, wherein the silica particles are sol-gel silica particles whose surfaces are treated with a trifunctional silane compound represented by formula (II) defined as

$$R^2Si(OR^3)_3 \qquad (II)$$

wherein $R^2$ represents a substituted or unsubstituted monovalent hydrocarbon group having 1 to 20 carbon atoms, and each $R^3$ independently represents a monovalent hydrocarbon group having 1 to 6 carbon atoms.

<3> The colloidal silica according to <1> or <2>, wherein the silica particles have a Z-average particle diameter of 5 to 300 nm, as determined by dynamic light scattering.

<4> The colloidal silica according to any one of <1> to <3>, wherein the dispersant is present in an amount of 10 to 3,000 ppm by mass based on the mass of the silica particles.

<5> The colloidal silica according to any one of <1> to <4>, comprising metal impurities at a concentration of 0 to 1 ppm by mass.

<6> The colloidal silica according to any one of <1> to <5>, comprising a hydrophilic organic solvent at a concentration of 0 to 1% by mass.

<7> The colloidal silica according to any one of <1> to <6>, wherein the colloidal silica has a solids concentration of 30 to 50% by mass.

<8> The colloidal silica according to any one of <1> to <7>, wherein the colloidal silica exhibits a kinematic viscosity of 1 to 100 $mm^2/s$ at 25°C.

<9> A chemical mechanical polishing liquid comprising the colloidal silica according to any one of <1> to <8>.

<10> A method for producing the colloidal silica according to any one of <1> and <3> to <8>, comprising the steps (A1), (A3') and (A4') of:

(A1) carrying out hydrolysis and condensation of

a tetrafunctional silane compound represented by formula (I) defined as $Si(OR^1)_4$ (I),
wherein each $R^1$ independently represents a monovalent hydrocarbon group having 1 to 6 carbon atoms,
a partially hydrolyzed condensate of the compound, or
a mixture thereof,

in a mixed liquid containing a hydrophilic organic solvent and water in the presence of a basic substance to produce a colloidal silica (A1);
(A3') adding at least one dispersant selected from 1,2-benzisothiazolin-3-one and isothiazolin-3-one to the colloidal silica (A1) obtained in the step (A1) to produce a dispersant-containing colloidal silica (A3'); and
(A4') replacing the hydrophilic organic solvent in a dispersion medium of the dispersant-containing colloidal silica (A3') obtained in the step (A3') with water.

<11> A method for producing the colloidal silica according to any one of <2> to <8>, comprising the steps (A1), (A2), (A3) and (A4) of:

(A1) carrying out hydrolysis and condensation of

a tetrafunctional silane compound represented by formula (I) defined as $Si(OR^1)_4$ (I),
wherein each $R^1$ independently represents a monovalent hydrocarbon group having 1 to 6 carbon atoms,
a partially hydrolyzed condensate of the compound, or
a mixture thereof,

in a mixed liquid containing a hydrophilic organic solvent and water in the presence of a basic substance to produce a colloidal silica;
(A2) adding

a trifunctional silane compound represented by formula (II) defined as $R^2Si(OR^3)_3$ (II),
wherein $R^2$ represents a substituted or unsubstituted monovalent hydrocarbon group having 1 to 20 carbon atoms and each $R^3$ independently represents a monovalent hydrocarbon group having 1 to 6 carbon atoms,
a partially hydrolyzed condensate of the compound, or
a mixture thereof,

into the colloidal silica (A1) obtained in the step (A1) to introduce $R^2SiO_{3/2}$ units on a surface of the colloidal silica to produce a surface-treated colloidal silica (A2);
(A3) adding at least one dispersant selected from 1,2-benzisothiazolin-3-one and isothiazolin-3-one to the surface-treated colloidal silica (A2) obtained in the step (A2) to produce a dispersant-containing surface-treated colloidal silica (A3); and
(A4) replacing the hydrophilic organic solvent in a dispersion medium of the dispersant-containing surface-treated colloidal silica (A3) obtained in the step (A3) with water.

<12> The method for producing the colloidal silica according to <11>, wherein, in the step (A2), the trifunctional silane compound represented by the formula (II), the partially hydrolyzed condensate, or the mixture thereof is added in an amount of 0.001 to 1 mole per mole of the $SiO_2$ unit in the colloidal silica (A1) obtained in the step (A1).

Advantageous Effects of Invention

The colloidal silica of the present invention maintains low viscosity even at high concentrations and exhibits minimal viscosity increase during long-term storage, thereby making it extremely useful as abrasive particles for, for example, polishing silicon wafers or the CMP process of semiconductor devices.

Description of Embodiments

The present invention will be described hereunder in greater detail.
The colloidal silica according to the present invention contains silica particles, at least one dispersant selected from 1,2-benzisothiazolin-3-one and isothiazolin-3-one, and water.

[Silica particles]

The silica particles contained in the colloidal silica of the present invention may be any type of silica particle which is not particularly limited so long as they are dispersible in water, but it is preferred that the particles be sol-gel silica particles, more preferably sol-gel silica particles whose surfaces are treated with a trifunctional silane compound represented by formula (II) defined as

$$R^2Si(OR^3)_3 \qquad (II)$$

wherein $R^2$ represents a substituted or unsubstituted monovalent hydrocarbon group having 1 to 20 carbon atoms, and each $R^3$ independently represents a monovalent hydrocarbon group having 1 to 6 carbon atoms.
In the above formula (II), $R^2$ is a monovalent hydrocarbon group having 1 to 20 carbon atoms, but it is preferred that $R^2$ be a monovalent hydrocarbon group having 1 to 10, particularly preferably 1 to 3 carbon atoms. Examples of the monovalent hydrocarbon group represented by $R^2$ include alkyl groups such as a methyl group, an ethyl group, a propyl group, an isopropyl group, a butyl group, a hexyl group, an octyl group, and a decyl group, of which particularly preferred are a methyl group, an ethyl group, and a propyl group. Additionally, a part or all of the hydrogen atoms in these monovalent hydrocarbon groups may be substituted with one or more halogen atoms, such as a fluorine atom, a chlorine atom, or a

bromine atom, preferably a fluorine atom.

In the above formula (II), $R^3$ is a monovalent hydrocarbon group having 1 to 6 carbon atoms, but it is preferred that $R^3$ be a monovalent hydrocarbon group having 1 to 4, particularly preferably 1 or 2 carbon atoms. Examples of the monovalent hydrocarbon group represented by $R^3$ include a methyl group, an ethyl group, a propyl group, a butyl group, and a phenyl group, among which preferred are a methyl group, an ethyl group, a propyl group, and a butyl group, and particularly preferred are a methyl group and an ethyl group.

Specific examples of the trifunctional silane compound represented by the above formula (II) include methyltrimethoxysilane, methyltriethoxysilane, ethyltrimethoxysilane, ethyltriethoxysilane, n-propyltrimethoxysilane, n-propyltriethoxysilane, i-propyltrimethoxysilane, i-propyltriethoxysilane, butyltrimethoxysilane, butyltriethoxysilane, hexyltrimethoxysilane, 3,3,3-trifluoropropyltrimethoxysilane, and (heptadecafluoro-1,1,2,2-tetrahydrodecyl)trimethoxysilane.

The silica particles contained in the colloidal silica of the present invention preferably have a Z-average particle diameter of 5 to 300 nm, more preferably of 10 to 150 nm, as determined by dynamic light scattering. For example, Zetasizer Ultra (manufactured by Malvern Panalytical Ltd) may be used to measure the Z-average particle diameter by dynamic light scattering.

[Dispersant]

The colloidal silica of the present invention contains at least one dispersant selected from 1,2-benzisothiazolin-3-one represented by the following structural formula (1) and isothiazolin-3-one represented by the following structural formula (2).

[Chem. 1]

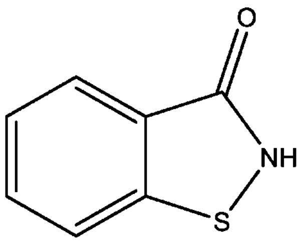

(1)

[Chem. 2]

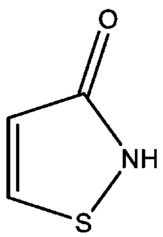

(2)

It can be generally considered that, as the concentration of silica particles in colloidal silica increases, the interparticle distance of the silica particles decreases, which promotes the formation of hydrogen bonding between silanol groups between silica particles and results in increased viscosity. However, it can also be considered that these dispersants work on the particles to disrupt these hydrogen bonds between silica particles, which prevents aggregation even at high silica concentrations and suppresses the viscosity increase of colloidal silica.

The dispersant is preferably present in an amount of 10 to 3,000 ppm by mass, more preferably of 500 to 2,000 ppm by mass, based on the mass of the silica particles.

[Dispersion medium]

The colloidal silica of the present invention contains water as a dispersion medium. The colloidal silica may also contain a hydrophilic organic solvent as a dispersion medium which is not water. Examples of such hydrophilic organic solvent include alcohols such as methanol, ethanol, 1-propanol, 2-propanol, and t-butanol; ethers such as tetrahydrofuran and dioxane; and glycols such as diethylene glycol and dipropylene glycol, of which preferred are alcohols.

The concentration of the hydrophilic organic solvent in the colloidal silica of the present invention is preferably 0 to

1% by mass, more preferably 0 to 100 ppm by mass, and particularly preferably 0 to 10 ppm by mass.

The total concentration of metal impurities such as sodium, potassium, iron, calcium, magnesium, titanium, nickel, chromium, and copper in the colloidal silica of the present invention is preferably 0 to 1 ppm by mass, more preferably 0 to 0.5 ppm by mass, relative to the total mass of the colloidal silica.

The solids concentration (concentration of silica particles) of the colloidal silica of the present invention is preferably 30% by mass or more, more preferably 30 to 50% by mass.

It is preferred that the colloidal silica of the present invention has a kinematic viscosity at 25°C of 1 to 100 $mm^2/s$, more preferably of 5 to 20 $mm^2/s$. The colloidal silica having a value within this range is suitable for use as an abrasive.

The colloidal silica of the present invention may contain any other ingredients as long as they do not impair the dispersion stability of the silica. For example, any one or more preservatives, disinfectants, and biocides may be added for the purpose of preventing bacterial growth. Examples of such compounds include hydrogen peroxide, ammonia, a quaternary ammonium hydroxide, a quaternary ammonium salt, ethylenediamine, glutaraldehyde, hydrogen peroxide, methyl p-hydroxybenzoate, isothiazolin-based compounds such as 2-methyl-4-isothiazolin-3-one, 5-chloro-2-methyl-4-isothiazolin-3-one, and 1,2-benzisothiazoline, and phenoxyethanol.

[Method for producing colloidal silica]

The colloidal silica of the present invention may be produced by a production method having the following steps (A1), (A3'), and (A4'), wherein

Step (A1) is a synthesizing step to produce a colloidal silica;
Step (A3') is a step of adding a dispersant; and
Step (A4') is a step of replacing the dispersion medium.

As a method for producing the colloidal silica of the present invention, preferable examples include a production method having the following steps (A1), (A2), (A3), and (A4), wherein

Step (A1) is a synthesizing step to produce a colloidal silica;
Step (A2) is a surface treatment step by a trifunctional silane compound;
Step (A3) is a step of adding a dispersant; and
Step (A4) is a step of replacing the dispersion medium.

Step (A1): Synthesizing step to produce colloidal silica

This step involves hydrolysis and condensation of a tetrafunctional silane compound represented by formula (I) shown below, its partially hydrolyzed condensate, or a mixture thereof, in a mixed liquid containing a hydrophilic organic solvent and water in the presence of a basic substance to produce a colloidal silica (A1),

$$Si(OR^1)_4 \qquad (I)$$

wherein each $R^1$ independently represents a monovalent hydrocarbon group having 1 to 6 carbon atoms.

In the formula (I), $R^1$ is a monovalent hydrocarbon group having 1 to 6 carbon atoms, but it is preferred that $R^1$ be a monovalent hydrocarbon group having 1 to 4, particularly preferably 1 or 2 carbon atoms. Examples of the monovalent hydrocarbon group represented by $R^1$ include a methyl group, an ethyl group, a propyl group, a butyl group, and a phenyl group, of which preferred are a methyl group, an ethyl group, a propyl group, and a butyl group, and particularly preferred are a methyl group and an ethyl group.

Examples of the tetrafunctional silane compound represented by the above formula (I) include tetraalkoxysilanes such as tetramethoxysilane, tetraethoxysilane, tetrapropoxysilane, and tetrabutoxysilane; and tetraphenoxysilane. Preferred are tetramethoxysilane, tetraethoxysilane, tetrapropoxysilane, and tetrabutoxysilane, and particularly preferred are tetramethoxysilane and tetraethoxysilane. Examples of the partially hydrolyzed condensates of the tetrafunctional silane compound represented by the above formula (I) include methyl silicates and ethyl silicates.

Examples of the basic substance include ammonia, dimethylamine, and diethylamine, of which preferred are ammonia and diethylamine, and particularly preferred is ammonia. These basic substances can be used in the form of being dissolved in water at a required amount.

The hydrophilic organic solvent may be any solvent as long as it solubilizes the compound represented by the above formula (I) and/or its partially hydrolyzed condensate as well as water, but examples thereof include alcohols such as methanol, ethanol, 1-propanol, 2-propanol, and t-butanol; ethers such as tetrahydrofuran and dioxane; and glycols such as diethylene glycol and dipropylene glycol, of which preferred are alcohols. An alcohol with a higher carbon number

tends to produce silica particles with a larger particle diameter. Accordingly, the type of alcohol may be selected based on the desired particle size of the resulting silica particles.

It is preferred that the amount of water used in this step be 0.5 to 5 mol, more preferably 0.6 to 2 mol, and particularly preferably 0.7 to 1 mol, per mol of total hydrocarbyloxy groups in the tetrafunctional silane compound represented by the above formula (I) and/or its partially hydrolyzed condensate. When the above-mentioned basic substance solubilized in water is used, the water used for solubilizing the basic substance is also included within the scope of "water" in this step.

It is preferred in terms of affinity between the silica particles and the mixed solvent as well as ease of production that the mass ratio of the hydrophilic organic solvent to water be 0.2 and 10 by mass, and more preferably 0.3 and 5 by mass.

It is preferred in terms of the size of the resulting silica particles that the amount of the basic substance be 0.01 to 2 mol, more preferably 0.03 to 0.5 mol, per mol of total hydrocarbyloxy groups in the tetrafunctional silane compound represented by the above formula (I) and/or its partially hydrolyzed condensate.

It is preferred that the hydrolytic condensation in this step be conducted under a reaction condition in which the reaction temperature is 20 to 120 °C and the reaction time is 1 to 8 hours, more preferably under a reaction condition in which the reaction temperature is 20 to 100 °C and the reaction time is 1 to 6 hours.

It is preferred that the colloidal silica (A1) obtained in the Step (A1) have silica particles at a concentration of 2 to 20% by mass, particularly preferably of 3 to 10% by mass.

Step (A2): Surface treatment step by trifunctional silane compound

This step is an optional step, and is a step of adding a trifunctional silane compound represented by formula (II) shown below, its partially hydrolyzed condensate, or a mixture thereof, into the colloidal silica (A1) obtained in the Step (A1) to introduce $R^2SiO_{3/2}$ units on the surface of the colloidal silica to thereby produce a surface-treated colloidal silica (A2),

$$R^2Si(OR^3)_3 \qquad (II)$$

wherein $R^2$ represents a substituted or unsubstituted monovalent hydrocarbon group having 1 to 20 carbon atoms and each $R^3$ independently represents a monovalent hydrocarbon group having 1 to 6 carbon atoms.

In the above formula (II), $R^2$ is a monovalent hydrocarbon group having 1 to 20 carbon atoms, but it is preferred that $R^2$ be a monovalent hydrocarbon group having 1 to 10, particularly preferably 1 to 3 carbon atoms. Examples of the monovalent hydrocarbon group represented by $R^2$ include alkyl groups such as a methyl group, an ethyl group, a propyl group, an isopropyl group, a butyl group, a hexyl group, an octyl group, and a decyl group, of which particularly preferred are a methyl group, an ethyl group, and a propyl group. Additionally, a part or all of the hydrogen atoms in these monovalent hydrocarbon groups may be substituted with one or more halogen atoms, such as a fluorine atom, a chlorine atom, or a bromine atom, preferably a fluorine atom.

In the above formula (II), $R^3$ is a monovalent hydrocarbon group having 1 to 6 carbon atoms, but it is preferred that $R^3$ be a monovalent hydrocarbon group having 1 to 4, particularly preferably 1 or 2 carbon atoms. Examples of the monovalent hydrocarbon group represented by $R^3$ include a methyl group, an ethyl group, a propyl group, a butyl group, and a phenyl group, among which preferred are a methyl group, an ethyl group, a propyl group, and a butyl group, and particularly preferred are a methyl group and an ethyl group.

Specific examples of the trifunctional silane compound represented by the above formula (II) include methyltrimethoxysilane, methyltriethoxysilane, ethyltrimethoxysilane, ethyltriethoxysilane, n-propyltrimethoxysilane, n-propyltriethoxysilane, i-propyltrimethoxysilane, i-propyltriethoxysilane, butyltrimethoxysilane, butyltriethoxysilane, hexyltrimethoxysilane, 3,3,3-trifluoropropyltrimethoxysilane, and (heptadecafluoro-1,1,2,2-tetrahydrodecyl)trimethoxysilane.

It is preferred that the trifunctional silane compound represented by the above formula (II) be used in an amount of 0.001 to 1 mol, more preferably of 0.01 to 0.1 mol, and particularly preferably of 0.01 to 0.05 mol, per mole of the $SiO_2$ unit (Si atom originating from the above formula (I)) of the colloidal silica obtained in the Step (A1).

This step (A2) involves adding a trifunctional silane compound represented by the above formula (II) to the colloidal silica (A1) obtained in the Step (A1) to perform a surface treatment reaction of the silica by the trifunctional silane compound under a reaction condition specified below. It is preferred that this step (A2) be performed under a reaction condition in which the reaction temperature is 40 to 60 °C and the reaction time is 1 to 10 hours, more preferably under a reaction condition in which the reaction temperature is 50 to 60 °C and the reaction time is 1 to 8 hours.

It is preferred that the surface-treated colloidal silica (A2) obtained in this step have a Z-average particle diameter of 5 to 300 nm, more preferably of 10 to 150 nm, as determined by dynamic light scattering.

Step (A3') and Step (A3): Step of adding dispersant

This step involves a step of adding at least one dispersant selected from 1,2-benzisothiazolin-3-one and isothiazolin-3-one to the colloidal silica (A1) obtained in the Step (A1) or to the surface-treated colloidal silica (A2) obtained in the Step (A2).
The dispersant is added in an amount preferably of 10 to 3,000 ppm by mass, more preferably of 500 to 2,000 ppm by mass, based on the mass of the silica particles.

Step (A4') and Step (A4): Step of replacing dispersion medium

This step involves a step of replacing the hydrophilic organic solvent, in the dispersion medium of the dispersant-containing colloidal silica (A3') obtained in the Step (A3') or the dispersant-containing surface-treated colloidal silica (A3) obtained in the Step (A3), with water. The hydrophilic organic solvent includes a hydrophilic organic solvent used in the Step (A1) and a volatile byproduct produced by condensation such as an alcohol.
Examples of the replacement method include: a heat concentration method, in which the dispersant-containing colloidal silica (A3') obtained in the Step (A3') or the dispersant-containing surface-treated colloidal silica (A3) obtained in the Step (A3) is heated and concentrated while adding water in an amount equal to or greater than the volume of distillate generated during heating; and a method such as ultrafiltration using a filter.
It is preferred from the viewpoint of the stability of colloidal silica that the amount of water added be 0.5 to 4 times, more preferably 1.8 to 3.0 times, by weight relative to the total amount of the hydrophilic organic solvent used and the alcohol produced.
Furthermore, when the dispersion medium substitution step is performed using a thermal concentration method so as to allow the finally obtained colloidal silica to have the above-mentioned desired solids concentration (i.e., silica particle concentration) or kinematic viscosity, the amount of hydrophilic organic solvent to be distilled off may be adjusted, the amount of water added during the substitution step of the dispersion medium may be appropriately controlled, or water may be added after the substitution step of the dispersion medium.

Examples

The present invention will be explained in detail below with reference to Working Examples and Comparative Examples. The following examples shall not be construed to limit the present invention in any way. The colloidal silica obtained in each of the Working Examples and Comparative Examples was evaluated as follows, and the results are as shown in Tables 1 and 2.

[Particle diameter (Z-Average particle diameter) and Polydispersity Index (PDI) by Dynamic Light Scattering]

A colloidal silica was diluted with ion-exchanged water to achieve a silica particle concentration of 0.1% by mass, and the resulting dispersion was subjected to ultrasonic irradiation for 10 minutes to measure the particle size distribution using Zetasizer Ultra (manufactured by Malvern Panalytical Ltd) to calculate the Z-average particle diameter and the PDI via dynamic light scattering. A PDI value of 0.1 or less is indicative of a highly monodisperse system.

[Solids concentration]

1.5 g of colloidal silica was accurately weighed and placed in an aluminum dish, dried at 105°C for 3 hours, and then the resultant residue (X) g was accurately weighed to calculate the silica solids concentration using the following formula:

$$\text{Silica solids concentration (\% by mass)} = (X/1.5) \times 100.$$

[Residual methanol content measurement]

A set of headspace gas chromatography measurement devices (HP6890 Series GC system, 5975 Mass Selective Detector, G1888 Network headspace Sampler, all manufactured by Agilent Technologies, Inc.) was used to analyze the vapor phase portion of the vial while heating at 60°C for 30 minutes to thereby measure the residual methanol content in the water-dispersed colloidal silica.

[Metal impurity content measurement]

An ICP optical emission spectrometer (Agilent 5110 ICP-OES, Agilent Technologies, Inc.) was used to quantify the metal elements to calculate the amount of metal impurities in the water-dispersed colloidal silica.

[Kinematic viscosity]

A Cannon-Fenske viscometer was used to measure the kinematic viscosity of the colloidal silica at 25°C.

In addition, 80 g of colloidal silica was placed in a 100 ml polyethylene bottle, sealed, stored at 60°C, and then the kinematic viscosity at 25°C was measured after 14, 30, and 60 days.

[Working Example 1]

Step (A1):

793.0 g of methanol, 32.1 g of ion-exchanged water, and 40.6 g of 28% aqueous ammonia were introduced into a 3-liter glass reactor equipped with a stirrer, a dropping funnel, and a thermometer, and mixed. This solution was adjusted to 46°C, and under continuous stirring, the dropwise addition of 646.5 g of tetramethoxysilane and 160.9 g of 5.5% aqueous ammonia was initiated simultaneously, with the former added over 6 hours and the latter over 4 hours. After completing the dropwise addition of tetramethoxysilane, stirring was continued for an additional 0.5 hour to promote hydrolysis and condensation, resulting in the formation of colloidal silica (A1). The silica particle concentration in this colloidal silica (A1) was 15.5% by mass.

Step (A2):

5.8 g of methyltrimethoxysilane (equivalent to 0.01 mole per mole of the $SiO_2$ unit in the colloidal silica) at 25°C was added to the colloidal silica (A1) obtained in the Step (A1), and the mixture was stirred at 55°C for 1 hour to produce a surface-treated colloidal silica (A2).

Step (A3):

To the surface-treated colloidal silica (A2) obtained in the Step (A2), 0.46 g of 1,2-benzisothiazolin-3-one (1,800 ppm by mass relative to the silica) was added while stirring the same at 25°C to produce a dispersant-containing surface-treated colloidal silica (A3).

Step (A4):

To the dispersant-containing surface-treated colloidal silica (A3) obtained in the Step (A3), 840 g of ion-exchanged water at 25°C was added. Subsequently, methanol was distilled off to achieve a content of solids of not less than 35% by mass, thereby producing 660 g of colloidal silica (i).

[Working Example 2]

690 g of colloidal silica (ii) was obtained in the same manner as in Working Example 1, except that, in the Step (A2) of Working Example 1, the amount of methyltrimethoxysilane added was modified to 28.9 g (equivalent to 0.05 mole per mole of the $SiO_2$ unit in the colloidal silica), and in the Step (A3) of Working Example 1, the amount of 1,2-benzisothiazolin-3-one added was modified to 0.15 g (600 ppm by mass relative to the silica).

[Working Example 3]

698 g of colloidal silica (iii) was obtained in the same manner as in Working Example 1, except that, in the Step (A2) of Working Example 1, the methyltrimethoxysilane was replaced with 6.9g of propyltrimethoxysilane (equivalent to 0.01 mole per mole of the $SiO_2$ unit in the colloidal silica)

[Working Example 4]

710 g of colloidal silica (iv) was obtained in the same manner as in Working Example 1, except that, in the Step (A2) of Working Example 1, the methyltrimethoxysilane was replaced with 9.2 g of 3,3,3-trifluoropropyltrimethoxysilane

(equivalent to 0.01 mole per mole of the $SiO_2$ unit in the colloidal silica), and in the Step (A3) of Working Example 1, the 1,2-benzisothiazolin-3-one was replaced with 0.46 g of isothiazolin-3-one (1,800 ppm by mass relative to the silica).

[Working Example 5]

655 g of colloidal silica (v) was obtained in the same manner as in Working Example 1, except that, in the Step (A1) of Working Example 1, the reaction temperature was changed to 38°C, and in the Step (A3) of Working Example 1, the amount of 1,2-benzisothiazolin-3-one added was modified to 0.15 g, with an additional 0.05 g of isothiazolin-3-one added (the combined amount of the two is equivalent to 800 ppm by mass relative to the silica).

[Working Example 6]

695 g of colloidal silica (vi) was obtained in the same manner as in Working Example 1, except that, in the Step (A1) of Working Example 1, the reaction temperature was changed to 30°C, and in the Step (A3) of Working Example 1, the amount of 1,2-benzisothiazolin-3-one added was modified to 0.51 g (2,000 ppm by mass relative to the silica).

[Working Example 7]

660 g of colloidal silica (vii) was obtained in the same manner as in Working Example 1, except that, in the Step (A1) of Working Example 1, the reaction temperature was changed to 20°C, and in the Step (A3) of Working Example 1, the 1,2-benzisothiazolin-3-one was replaced with 0.51 g of isothiazolin-3-one (2,000 ppm by mass relative to the silica).

[Working Example 8]

• Step (A1)

793.0 g of methanol, 32.1 g of ion-exchanged water, and 40.6 g of 28% aqueous ammonia were introduced into a 3-liter glass reactor equipped with a stirrer, a dropping funnel, and a thermometer, and mixed. This solution was adjusted to 46°C, and under continuous stirring, the dropwise addition of 646.5 g of tetramethoxysilane and 160.9 g of 5.5% aqueous ammonia was initiated simultaneously, with the former added over 6 hours and the latter over 4 hours. After completing the dropwise addition of tetramethoxysilane, stirring was continued for an additional 0.5 hour to promote hydrolysis and condensation, resulting in the formation of colloidal silica (A1). The silica particle concentration in this colloidal silica (A1) was 15.5% by mass.

Step (A3'):

To the colloidal silica (A1) obtained in the Step (A1), 0.46 g of 1,2-benzisothiazolin-3-one (1,800 ppm by mass relative to the silica) was added while stirring the same at 25°C to produce a dispersant-containing colloidal silica (A3').

Step (A4'):

To the dispersant-containing colloidal silica (A3') obtained in the Step (A3'), 840 g of ion-exchanged water at 25°C was added. Subsequently, methanol was distilled off to achieve a content of solids of not less than 35% by mass, thereby producing 659 g of colloidal silica (viii).

[Comparative Example 1]

655 g of colloidal silica (ix) was obtained in the same manner as in Working Example 8, except that the Step (A3') was not carried out.

[Comparative Example 2]

675 g of colloidal silica (x) was obtained in the same manner as in Working Example 8, except that, in the Step (A1) of Working Example 8, the reaction temperature was changed to 20°C and the Step (A3') of Working Example 8 was not carried out.

[Comparative Example 3]

690 g of colloidal silica (xi) was obtained in the same manner as in Working Example 8, except that, in the Step (A3') of Working Example 8, the 1,2-benzisothiazolin-3-one was replaced with 0.17 g of ammonium benzoate (600 ppm by mass relative to the silica).

[Comparative Example 4]

695 g of colloidal silica (xii) was obtained in the same manner as in Working Example 8, except that, in the Step (A3') of Working Example 8, the 1,2-benzisothiazolin-3-one was replaced with 0.09 g of citric acid (300 ppm by mass relative to the silica).

[Comparative Example 5]

680 g of colloidal silica (xiii) was obtained in the same manner as in Working Example 1, except that, in the Step (A3) of Working Example 1, the 1,2-benzisothiazolin-3-one was replaced with 0.46 g of 2-methyl-4-isothiazolin-3-one (1,800 ppm by mass relative to the silica).

[Table 1]

| | | Working Examples | | | | | | | |
|---|---|---|---|---|---|---|---|---|---|
| | | 1 | 2 | 3 | 4 | 5 | 6 | 7 | 8 |
| Colloidal silica | | i | ii | iii | iv | v | vi | vii | viii |
| Particle diameter (nm) | | 35 | 35 | 35 | 35 | 55 | 70 | 95 | 35 |
| PDI | | 0.079 | 0.081 | 0.085 | 0.090 | 0.073 | 0.061 | 0.041 | 0.080 |
| Solids concentration (%) | | 39.2 | 39.1 | 37.2 | 36.9 | 39.4 | 37.2 | 39.2 | 38.7 |
| Residual methanol content (ppm) | | Below 10 | Below 10 | Below 10 | Below 10 | Below 10 | Below 10 | Below 10 | Below 10 |
| Metal impurity content (ppm) | | Below 0.1 | Below 0.1 | Below 0.1 | Below 0.1 | Below 0.1 | Below 0.1 | Below 0.1 | Below 0.1 |
| Kinematic viscosity ($mm^2/s$) | Initial time point | 12.1 | 29.0 | 11.5 | 10.1 | 9.3 | 6.1 | 5.2 | 13.6 |
| | 60°C, after 14 days | 13.0 | 29.3 | 11.5 | 10.3 | 9.3 | 6.1 | 5.2 | 14.2 |
| | 60°C, after 30 days | 13.1 | 29.3 | 11.7 | 10.2 | 9.5 | 6.2 | 5.5 | 14.5 |
| | 60°C, after 60 days | 13.2 | 30.0 | 11.7 | 10.5 | 9.8 | 6.2 | 5.6 | 14.8 |

[Table 2]

| | Comparative Examples | | | | |
|---|---|---|---|---|---|
| | 1 | 2 | 3 | 4 | 5 |
| Colloidal silica | ix | x | xi | xii | xiii |
| Particle diameter (nm) | 35 | 95 | 35 | 35 | 35 |
| PDI | 0.078 | 0.042 | 0.078 | 0.076 | 0.076 |
| Solids concentration (%) | 39.0 | 37.8 | 37.0 | 36.7 | 38.0 |
| Residual methanol content (ppm) | 450 | 880 | Below 10 | Below 10 | 880 |
| Metal impurity content (ppm) | Below 0.1 | Below 0.1 | Below 0.1 | Below 0.1 | Below 0.1 |

(continued)

| | | Comparative Examples | | | | |
|---|---|---|---|---|---|---|
| | | 1 | 2 | 3 | 4 | 5 |
| Kinematic viscosity ($mm^2/s$) | Initial time point | Above 800 | Above 800 | 560 | 330 | Above 800 |
| | 60°C, after 14 days | - | - | 600 | 500 | - |
| | 60°C, after 30 days | - | - | Above 800 | Above 800 | - |
| | 60°C, after 60 days | - | - | - | - | - |

The above results demonstrate that the colloidal silica produced by the production method according to the present invention maintains low viscosity even at high concentration and exhibits minimal viscosity increase during long-term storage.

Industrial Applicability

The colloidal silica of the present invention maintains low viscosity even at high concentrations and exhibits minimal viscosity increase during long-term storage, thereby making it suitable for use as abrasive particles for, for example, polishing silicon wafers or the CMP process of semiconductor devices.

## Claims

**1.** A colloidal silica comprising:

silica particles;
at least one dispersant selected from 1,2-benzisothiazolin-3-one and isothiazolin-3-one; and
water.

**2.** The colloidal silica according to claim 1, wherein the silica particles are sol-gel silica particles whose surfaces are treated with a trifunctional silane compound represented by formula (II) defined as

$$R^2Si(OR^3)_3 \qquad (II)$$

wherein $R^2$ represents a substituted or unsubstituted monovalent hydrocarbon group having 1 to 20 carbon atoms, and each $R^3$ independently represents a monovalent hydrocarbon group having 1 to 6 carbon atoms.

**3.** The colloidal silica according to claim 1, wherein the silica particles have a Z-average particle diameter of 5 to 300 nm, as determined by dynamic light scattering.

**4.** The colloidal silica according to claim 1, wherein the dispersant is present in an amount of 10 to 3,000 ppm by mass based on the mass of the silica particles.

**5.** The colloidal silica according to claim 1, comprising metal impurities at a concentration of 0 to 1 ppm by mass.

**6.** The colloidal silica according to claim 1, comprising a hydrophilic organic solvent at a concentration of 0 to 1% by mass.

**7.** The colloidal silica according to claim 1, wherein the colloidal silica has a solids concentration of 30 to 50% by mass.

**8.** The colloidal silica according to claim 1, wherein the colloidal silica exhibits a kinematic viscosity of 1 to 100 $mm^2/s$ at 25°C.

**9.** A chemical mechanical polishing liquid comprising the colloidal silica according to any one of claims 1 to 8.

**10.** A method for producing the colloidal silica according to claim 1, comprising the steps (A1), (A3') and (A4') of:

(A1) carrying out hydrolysis and condensation of

a tetrafunctional silane compound represented by formula (I) defined as $Si(OR^1)_4$ (I),
wherein each $R^1$ independently represents a monovalent hydrocarbon group having 1 to 6 carbon atoms,
a partially hydrolyzed condensate of the compound, or
a mixture thereof,

in a mixed liquid containing a hydrophilic organic solvent and water in the presence of a basic substance to produce a colloidal silica (A1);
(A3') adding at least one dispersant selected from 1,2-benzisothiazolin-3-one and isothiazolin-3-one to the colloidal silica (A1) obtained in the step (A1) to produce a dispersant-containing colloidal silica (A3'); and
(A4') replacing the hydrophilic organic solvent in a dispersion medium of the dispersant-containing colloidal silica (A3') obtained in the step (A3') with water.

**11.** A method for producing the colloidal silica according to claim 2, comprising the steps (A1), (A2), (A3) and (A4) of:

(A1) carrying out hydrolysis and condensation of

a tetrafunctional silane compound represented by formula (I) defined as $Si(OR^1)_4$ (I),
wherein each $R^1$ independently represents a monovalent hydrocarbon group having 1 to 6 carbon atoms,
a partially hydrolyzed condensate of the compound, or
a mixture thereof,

in a mixed liquid containing a hydrophilic organic solvent and water in the presence of a basic substance to produce a colloidal silica;
(A2) adding

a trifunctional silane compound represented by formula (II) defined as $R^2Si(OR^3)_3$ (II),
wherein $R^2$ represents a substituted or unsubstituted monovalent hydrocarbon group having 1 to 20 carbon atoms and each $R^3$ independently represents a monovalent hydrocarbon group having 1 to 6 carbon atoms,
a partially hydrolyzed condensate of the compound, or
a mixture thereof,

into the colloidal silica (A1) obtained in the step (A1) to introduce $R^2SiO_{3/2}$ units on a surface of the colloidal silica to produce a surface-treated colloidal silica (A2);
(A3) adding at least one dispersant selected from 1,2-benzisothiazolin-3-one and isothiazolin-3-one to the surface-treated colloidal silica (A2) obtained in the step (A2) to produce a dispersant-containing surface-treated colloidal silica (A3); and
(A4) replacing the hydrophilic organic solvent in a dispersion medium of the dispersant-containing surface-treated colloidal silica (A3) obtained in the step (A3) with water.

**12.** The method for producing the colloidal silica according to claim 11, wherein, in the step (A2), the trifunctional silane compound represented by the formula (II), the partially hydrolyzed condensate, or the mixture thereof is added in an amount of 0.001 to 1 mole per mole of the $SiO_2$ unit in the colloidal silica (A1) obtained in the step (A1).

**INTERNATIONAL SEARCH REPORT**

International application No. **PCT/JP2024/018642**

**A. CLASSIFICATION OF SUBJECT MATTER**

***C01B 33/141***(2006.01)i; ***B01J 13/00***(2006.01)i; ***C07D 275/03***(2006.01)i; ***C07D 275/04***(2006.01)i; ***C09K 3/14***(2006.01)i; ***H01L 21/304***(2006.01)i

FI: C01B33/141; B01J13/00 B; C09K3/14 550D; C09K3/14 550Z; H01L21/304 622B; C07D275/03; C07D275/04

According to International Patent Classification (IPC) or to both national classification and IPC

**B. FIELDS SEARCHED**

Minimum documentation searched (classification system followed by classification symbols)

C01B33/141; B01J13/00; C07D275/03; C07D275/04; C09K3/14; H01L21/304

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2021-50268 A (FUJIMI INC.) 01 April 2021 (2021-04-01) claims, paragraphs [0002], [0015]-[0024], [0077]-[0081], [0090] | 1, 3, 5, 6, 8, 9 |
| Y | | 2, 10-12 |
| A | | 4, 7 |
| Y | JP 2023-5414 A (SHIN-ETSU CHEMICAL CO., LTD.) 18 January 2023 (2023-01-18) claims , paragraph [0011] | 2, 10-12 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **31 July 2024** | **13 August 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/018642**

| Patent document cited in search report | Publication date (day/month/year) | Patent family member(s) | Publication date (day/month/year) |
|---|---|---|---|
| JP 2021-50268 A | 01 April 2021 | (Family: none) | |
| JP 2023-5414 A | 18 January 2023 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005162533 A
- JP 5270344 B
- JP 2019172558 A
- JP 2023005414 A